# EUROPEAN PATENT SPECIFICATION

(11) **EP 1 795 171 B1**
(45) Date of publication and mention of the grant of the patent: **19.11.2014**
(21) Application number: 05111859.4
(22) Date of filing: 08.12.2005
(51) Int. Cl.: A61K 6/06, A61K 6/083, A61L 24/00, A61L 27/42, C04B 22/10, C04B 22/08, C04B 22/14, C04B 22/12, C04B 22/16, C04B 12/04, C04B 28/18

(54) **Powdered CBC system with improved reaction feature**
System mit verbesserten Reaktion-Eigenschaften aus einer pulverförmigen, chemisch gebundenen Keramik
Système ayant des propriétés de réaction améliorées et constitué par une poudre de matériau céramique à liage chimique

(43) Date of publication of application: 13.06.2007
(73) Proprietor: Doxa AB, 754 51 Uppsala (SE)
(72) Inventor: Hermansson, Leif, 26042 Mölle (SE); Engqvist, Håkan, 741 44 Knivsta (SE)
(74) Representative: Brann AB

(56) References cited:
- EP-A- 0 286 396
- WO-A-03/082765
- WO-A-2005/039508
- US-A- 5 624 489
- US-A- 5 961 712

## Description

### TECHNICAL FIELD

For medical devices which are formed *in vivo* the biocompatibility profile, especially during the initial reactive stage, is of specific importance. Many chemically bonded ceramics, and especially aluminate and silicate ceramics, which are formed as a result of an acid-base reaction, exhibit a high pH-range during the first hours. The present invention is directed to such compositions exhibiting a lowered pH range during the initial reaction period. The composition of the invention is especially intended for endodontic and orthopaedic applications, and also for soft tissue applications.

### STATE OF THE ART AND PROBLEM

Implants that are to interact with the human body should advantageously be composed of materials having a good biocompatibility and bioactivity. An example of such a material is the class known as "chemically bonded ceramics" (CBC). The hardening of such materials is accomplished by chemical reaction with water, involving formation of stable hydrates, precipitation and crystallization thereof, which reaction takes place already at ambient temperature, such as for example in *in vivo* conditions. Chemically bonded ceramics have been shown to be bioactive in the sense that the materials in contact with phosphate solution (both *in vitro* and *in vivo*) form apatite in the contact zone between the biomaterial and the tissue.

Examples of such materials are the high strength CBC materials, which are based on Ca-aluminates and/or Ca-silicates, such as those described in SE463493, SE502987, WO 2000/021489, WO 2001/076534, WO 2001/076535, WO 2004/037215, WO 2004/00239 and WO 2003/041662. Said materials have been proposed for use in dental applications.

It would be desirable to be able to use such materials also in endodontic and orthopaedic applications.

Accordingly, it is an object of the present invention to improve the characteristics of the above materials in order to make them better suited for use in endodontic and orthopaedic applications.

For a CBC material producing an initial pH of ≥ 10 on hydration the problem has been solved by means of the characterizing features of claim 1, according to which a solid phase buffer component is included in the CBC material forming powder composition.

### SUMMARY OF THE INVENTION

The present inventors have found the inherently high pH value produced on hydration of many of the above prior art materials to make them less suited for use in endodontic and orthopaedic applications. As an example, most of the above Ca-aluminates and Ca-silicates hydrate in the basic pH-range of 10.5-12.5. This is especially believed to be a problem when said materials are to be used in applications other than dental applications and coatings on implants requiring larger amounts of the material.

The high pH value has been found to be connected with the initial hydration reaction of the material (see Tables 1 and 2 for Ca-aluminate and Ca-silicate, respectively). After the initial reaction the subsequent diffusion controlled reaction is controlled with regard to pH by the body liquid buffer system. Accordingly, after the initial hydration reaction, which has been found to be intense during the first hours up to one day, and especially during the first 4 hours, the use of Ca-aluminate and Ca-silicate systems should no longer be problematic.

The present inventors have now found that the initially high pH value produced on hydration can be successfully controlled to be within a reduced pH range already after 1 hour of hydration reaction by including a solid phase buffer component in the powder system forming the CBC material. Such powder system is provided for in claim 1. The system is especially intended for endodontic and orthopaedic applications.

According to the present invention the period of time during which an alkaline CBC system produces a critically high pH value *in vivo* can be markedly reduced.

Therefore, the inventive CBC system containing the buffer component will to a lesser extent affect the endogenous buffer system than the prior art alkaline CBC system not having a buffer component, thus making the inventive system more tolerable *in vivo.*

The use of a buffer component in the solid state according to the invention allows for a very effective buffering action.

Also, the use of buffer components exhibiting anions which can form part of the structure of chemically bound material makes the buffering component a more integral part of the system and allows for larger amounts of the buffer component to be used.

In addition to endodontic and orthopaedic applications, the material of the invention is also believed to be suitable for soft tissue applications by virtue of the reduced pH during the initial hydration reaction.

Further features and advantages of the present invention will be evident from the following detailed description and dependent claims.

The system of the invention also allows for incorporation of pH sensitive drugs and/or bone growth promoting agents, prophylactically and/or diagnostically active agents to be released subsequently by the chemically bound ceramic biomaterial *in vivo.* Examples of such drugs include therapeutic agents for many areas, e.g. pain relief, anaesthetics, anti-infective agents and anti-inflammatory drugs, drugs for treatment of immunological disorders, hormonal disruption, clotting behaviour, cancer, and combinations thereof. In these cases the agents can be contained in the porous implant, precursor or implant material or as separate porous granules.

### BRIEF DESCRIPTION OF THE ATTACHED DRAWINGS

Figure 1 shows the pH change over time of a system according to the invention producing a rapid controlled pH change.
Figure 2 shows a plot of the pH as a function of time for a controlled and delayed pH reduction after a given period of time to optimize the hydration during the first 30-60 minutes, and thereafter a substantially reduced pH to a more biocompatible range.

### DETAILED DESCRIPTION OF THE INVENTION

The internal buffer system is composed of a buffering salt of mono-, di-, tri- and/or tetravalent acids. The salts are typically salts of mineral acids of the type [Me:H:Anion], wherein:
Me = Na, K, Mg, Ca ;
Anion = CO₃²⁻, PO₄³⁻, SO₃²⁻, SO₄²⁻, SiO₄⁴⁻ or fluoride; and
H = one or more hydrogen ions.

In one embodiment the CBC material of the invention is combined with an added acid as dry component in inert particle porosities for a rapid change early in the hydration process, such as improved early-age mechanical properties, as described in WO 2005/039508.

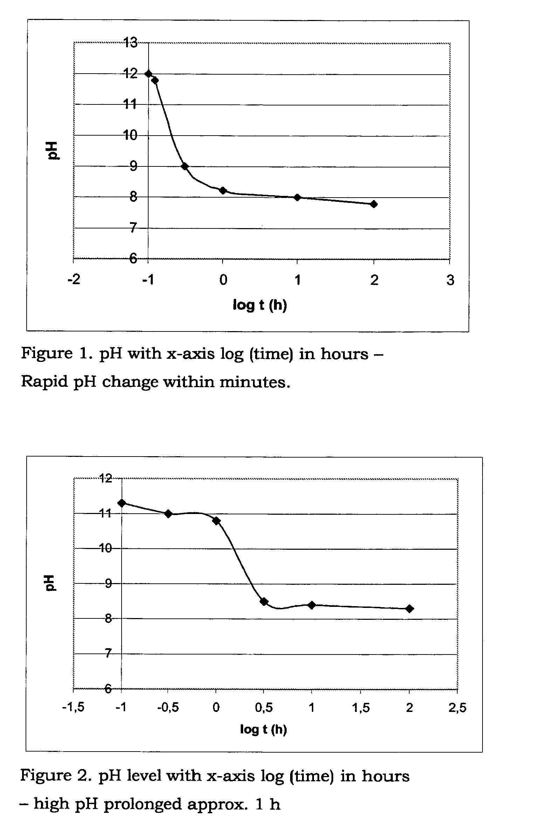

The pH change over time is presented in Figure 1. Another advantage of a controlled pH reduction after a given period of time according to the invention is the possibility to optimize the hydration during the first 30-60 minutes, and thereafter obtaining a substantially reduced pH to a more biocompatible range. See the pH vs. time plot in Figure 2. In such embodiment, in order to achieve an optimized hydration during the first 30-60 minutes, the buffering salt can be impregnated in porous granules of the component forming the chemically bonded ceramic material and/or in an additional porous inert phase. The diffusion of the buffer salt into the surrounding media will thereby be delayed.

According to the present invention the initial hydration reaction of very alkaline systems, such as Ca-aluminate and Ca-silicate (with an initial pH value on hydration of 11-12.5), can be effectively controlled to be in a reduced pH range of 7.5-10 already after 1 hour of hydration reaction, preferably within the range 7.5 to 9.5, and more preferably within the range of 7.5 to 9.0, by the use of an internal buffer system added to the powdered biomaterial of CBC type used. The internal buffer system is composed of a buffering salt of mono-, di-, tri- and/or tetravalent mineral acids. The salts are typically acidic salts of the type [Me:H:Anion], wherein:
Me = Na, K, Mg, Ca ;
Anion = CO₃²⁻, PO₄³⁻, SO₃²⁻, SO₄²⁻, SiO₄⁴⁻ and fluoride; and
H = one or more hydrogen ions.

Very attractive are the acid salts forming the ions HF₂-, HCO₃-, HPO₄²⁻ and H₂PO₄- in aqueous solutions.

Examples of most suitable salts for use as the buffer are: KHF₂, NaHCO₃, Mg(Ca)HPO₄, Na₂HPO₄ and NaH₂PO₄.

The pH development has been found to depend on three major material sources; the solid raw material (CBC), the general hydration liquid used, and the body liquid encountered after implantation. Especially effective with high buffering capacity are the solid buffers according to the present invention. The buffering capacity can be expressed as mM OH-. The capacity is as high as 100 mM, corresponding to a pH change from approximately 13 to near neutral, i.e. pH 7-8. The buffering capacity of body liquids are considerably lower due to two aspects; the ion buffering capacity expressed as mM is considerably lower, and secondly the body liquids are external and thus pH reduction is diffusion-time restricted, as generally described in Ceramics, Cells and Tissues, Ed Ravaglioli and Krajewski, pp 200-201, published by IS-TEC-CNR-Faenza, May 2005. The buffering salts are preferably added in solid form to the ceramic powder. However, the salts could also be dissolved in the hydrating liquid, in which case the required buffer salt could be added to the hydration liquid only, or to both liquid and powder in any desired ratio.

The skilled person having read the present disclosure can readily establish the necessary amount of a given buffer salt to be added to a given amount of the composition in order to achieve a desired pH value on hydration, e.g. merely by routine pH measurements. With reference to Table 3 it can be seen that the necessary amount is dependent upon the specific CBC material used. For example, the silicate material is more alkaline than the aluminate material and will consequently require a larger amount of any given buffer salt.

In general the amount of the buffer is selected so as to obtain a pH value within the range of 7.5 to 10 after 1 hour of hydration reaction, preferably within the range 7.5 to 9.5, and more preferably within the range of 7.5 to 9.0.

The amount of the buffer should not exceed 30% by volume of the total volume of the constituents of the powder system in the dry state, since otherwise the mechanical properties of the bound material will be negatively effected. On the other hand, an amount of less than 3% by volume of any buffer salt will not produce a sufficient pH controlling effect.

With reference to Tables 1 and 2 it can be seen that the pH in phosphate buffer solution (i.e. a simulated body fluid), declines more rapidly than in water. A volume of 3% of the acid buffer salt of the invention is generally sufficient to produce a pH reduction at 1 hour of hydration of at least 0.5.

With reference to the total hydrating system, i.e. the powdery constituents together with hydration liquid, a preferred amount the acidic buffer salt is within the range of 5-20% by weight, and more preferably 5-10% by weight.

Suitable Ca-aluminate and/or Ca-silicate systems for implants which can be used in the present invention are described in e.g. Swedish patent applications Nos.: SE 0200637-7, SE 0203223-3 and SE 0203324-1. Additives which can be used are described in SE 463493, SE 502987, WO 00/21489, WO 01/76534, WO 01/76535, WO 2004/037215 and WO 2003/041662, the relevant contents of which are incorporated herein by reference.

As disclosed in WO 2005/039508, in order to improve the controllability of the composition's initial viscosity and consistency, and early-age properties (initial strength, pore closure, translucency and early obtained bioactivity), a polycarboxylic acid or a copolymer or a salt or an ester thereof having a molecular weight of 100-250,000, preferably 1000-100,000 could be added to the powder. The amount of the acid or the copolymer, salt or ester thereof must however be selected so as not interfere with the buffering action of the salt, or compromise the desired mechanical properties of the hardened material. A suitable amount is believed to be within the range of 1-15 % and preferably 2-5 % by weight, based on the powdered material including any dry additives.

### EXAMPLES

Ca-aluminate (CA) and Ca-silicate (C₃S) were synthesized at 1410 and 1380 °C, respectively, in a conventional sintering furnace for 6 h. The materials were crushed and thereafter milled for 72 h in a rotating mill using ceramics containers and with the milling media of Silicon nitride balls (15 mm in diameter) and iso-propanol as liquid. After thin film evaporation the powder was pressed to small pellets. The pellet dimensions were: length, 1 = 6 mm; and diameter, d = 4 mm. The compact density was approximately 59%.

In tables 1 and 2 pH development for a pure Ca-aluminate and a pure Ca-silicate phase (0-30 days with exchange of liquid after each test time), respectively, are shown. In table 3 pH development for the systems in examples (tables 1 and 2) with different amounts of three different internal buffer systems added (KHF₂, NaHCO₃ and MgHPO₄ and NaH₂PO₄) according to table 3 below, are presented. The amount of added internal buffer was a = 5, b = 10 and c = 20 wt-% of the weight of the hydrating system used.

**Table 1. The pure system Ca-aluminate and pH development in water (W) and phosphate buffer solution (PBS).**

| Medium | Time zero | 1h | 4h | 24h | 1 week | 1 month |
|---|---|---|---|---|---|---|
| W | 11.5 | 11.3 | 11.0 | 9.4 | 8.8 | 8.4 |
| PBS | 11.5 | 10.2 | 9.5 | 8.0 | 7.8 | 7.8 |

**Table 2. The pure system Ca-silicate and pH development in water (W) and phosphate buffer solution (PBS).**

| Medium | Time zero | 1h | 4h | 24h | 1 week | 1 month |
|---|---|---|---|---|---|---|
| W | 12.4 | 12.0 | 11.8 | 10.5 | 9.0 | 9.0 |
| PBS | 12.4 | 11.5 | 10.5 | 9.5 | 9.0 | 9.0 |

**Table 3. Ca-aluminate (CA) and two different types of internal buffer components in phosphate buffer solution, (1-3 = KHF₂, 4-6 = , NaHCO₃) and Ca-silicate (C₃S) and two different types of internal buffer components in water (7-9 = and MgHPO₄ and 10-12 = and Na H₂PO₄).**

| Type | 1 a=5 | 2 b=10 | 3 b=20 | 4 a=5 | 5 b=10 | 6 c=20 | 7 a=5 | 8 b=10 | 9 c=20 | 10 a=5 | 11 b=10 | 12 c=20 |
|---|---|---|---|---|---|---|---|---|---|---|---|---|
| After | | | | | | | | | | | | |
| 5min¹ | 11.5 | 11.5 | 10.5 | 11.5 | 11.0 | 10.0 | 12.3 | 11.8 | 11.5 | 12.0 | 11.2 | 10.2 |
| 5 min² | 11.3 | 11.3 | 10.0 | 11.0 | 10.5 | 9.5 | 12.0 | 11.5 | 11.2 | 11.5 | 10.5 | 9.8 |
| 1h | 9.5 | 9.3 | 9.0 | 9.5 | 9.0 | 9.0 | 10.0 | 10.0 | 9.8 | 9.0 | 8.5 | 8.0 |
| 4h | 9.0 | 9.0 | 8.5 | 8.5 | 8.0 | 8.0 | 10.0 | 10.0 | 9.7 | 8.5 | 8.0 | 8.0 |
| 24h | 8.0 | 8.0 | 7.8 | 8.5 | 8.0 | 8.0 | 9.5 | 9.0 | 9.0 | 8.0 | 8.0 | 8.0 |
| 1 w | 8.0 | 8.0 | 7.8 | 8.5 | 8.0 | 8.0 | 8.5 | 8.0 | 8.0 | 8.0 | 8.0 | 7.7 |
| 1 m | 8.0 | 8.0 | 7.8 | 8.0 | 8.0 | 7.5 | 8.0 | 8.0 | 8.0 | 8.0 | 8.0 | 7.7 |

| | | | | | | | | | | | | |
|---|---|---|---|---|---|---|---|---|---|---|---|---|
| ¹ After 5 min means 5 min after start of mixing the powder and liquid. ² 5 min means 5 min after mixing is completed. The mixing takes approx. 2 minutes. | | | | | | | | | | | | |

## Claims

1. Powdered composition forming on hydration thereof a chemically bonded ceramic biomaterial for use in endodontic, orthopaedic, and soft tissue applications, including (a) an alkaline component that on hydration thereof forms a chemically bonded ceramic biomaterial and produces an initial pH of ≥ 10, **characterised in that** the composition also includes (b) a solid buffering salt of the type [Me:H:Anion] of a mono-, di-, tri- or tetravalent mineral acid in an amount of 3-30% by volume, wherein:
Me = Na, K, Mg, Ca;
Anion = CO₃²⁻, PO₄³⁻, SO₃²⁻, SO₄²⁻, SiO₄⁴⁻ or fluoride; and
H = one or more hydrogen ions.

2. Powdered composition of claim 1, **characterised in that** the solid buffer phase produces the ions selected from HF₂⁻, HCO₃⁻, HPO₄²⁻ and H₂PO₄⁻.

3. Powdered composition of claim 1 or 2, **characterised in that** component (a) exhibits an initial pH value of ≥ 11 on hydration thereof.

4. Powdered composition of any of the previous claims, **characterised in that** component (a) is composed of aluminates and/or silicates.

5. Powdered composition of any of the previous claims, **characterised in that** amount of (b) is selected such that a pH of 7.5-10, preferably 7.5-9.5, and more preferably 7.5-9 is obtained after 1 hour of hydration of composition.

6. Powdered composition of any of the previous claims, **characterised in that** the buffering salt (b) is impregnated in porous granules of component (a) and/or in an additional porous inert phase.

7. Powdered composition of any of the previous claims, **characterised in** also comprising a conventional additive.

8. Powdered composition according to any of the previous claims, further containing pH sensitive drugs and/or bone growth promoting agents, prophylactically and/or diagnostically active agents to be released subsequently by the chemically bound ceramic biomaterial *in vivo,* e.g. therapeutic agents for pain relief, anaesthetics, anti-infective agents and anti-inflammatory drugs, drugs for treatment of immunological disorders, hormonal disruption, clotting behaviour, cancer, and combinations thereof.

9. Composition, forming on hydration thereof a chemically bonded ceramic biomaterial for use in endodontic, orthopaedic, and soft tissue applications, including (a) an alkaline component that on hydration thereof forms a chemically bonded ceramic biomaterial and produces an initial pH of ≥ 10, and (c) hydration liquid, **characterised in that** the composition also includes (b) a buffering salt of the type [Me:H:Anion] of a mono-, di-, tri- or tetravalent mineral acid in an amount of 3-30% by volume based on the total volume of the constituents in dry state, or 5-20% by weight of the hydrating system,
wherein:
Me = Na, K, Mg, Ca;
Anion = CO₃²⁻, PO₄³⁻, SO₃²⁻, SO₄²⁻, SiO₄⁴⁻ or fluoride; and
H = one or more hydrogen ions.

10. Method of preparing the composition of claim 9, comprising the step of mixing (a) an alkaline component that on hydration thereof forms a chemically bonded ceramic biomaterial and produces an initial pH of ≥ 10, and (c) hydration liquid, **characterised in that** a buffering salt of the type [Me:H:Anion] of a mono-, di-, tri- or tetravalent mineral acid in an amount of 3-30% by volume based on the total volume of the constituents in dry state, or 5-20% by weight of the hydrating system, wherein:
Me = Na, K, Mg, Ca;
Anion = CO₃²⁻, PO₄³⁻, SO₃²⁻, SO₄²⁻, SiO₄⁴⁻ or fluoride; and
H = one or more hydrogen ions
is dissolved in the hydration liquid (c) and/or added to compound (a).

## Patentansprüche

1. Pulverförmige Zusammensetzung, welche nach Hydratation davon ein chemisch gebundenes keramisches Biomaterial zur Verwendung bei endodontischen, orthopädischen und Weichgewebe-Anwendungen bildet, umfassend (a) eine alkalische Komponente, welche nach Hydratation davon ein chemisch gebundenes keramisches Biomaterial bildet und einen anfänglichen pH von ≥ 10 erzeugt, **dadurch gekennzeichnet, dass** die Zusammensetzung auch (b) ein festes Puffersalz vom Typ [Me:H:Anion] einer ein-, zwei-, drei- oder vierwertigen Mineralsäure in einer Menge von 3-30 Vol.-% enthält,
wobei:
Me = Na, K, Mg, Ca;
Anion = CO₃²⁻, PO₄³⁻, SO₃²⁻, SO₄²⁻, SiO₄⁴⁻ oder Fluorid und
H = ein oder mehrere Wasserstoffionen.

2. Pulverförmige Zusammensetzung nach Anspruch 1, **dadurch gekennzeichnet, dass** die feste Pufferphase die Ionen, ausgewählt aus HF₂⁻, HCO₃⁻, HPO₄²⁻ und H₂PO₄⁻, erzeugt.

3. Pulverförmige Zusammensetzung nach Anspruch 1 oder 2, **dadurch gekennzeichnet, dass** Komponente (a) einen anfänglichen pH-Wert von ≥ 11 nach Hydratation davon aufweist.

4. Pulverförmige Zusammensetzung nach einem der vorhergehenden Ansprüche, **dadurch gekennzeichnet, dass** Komponente (a) aus Aluminaten und/oder Silikaten zusammengesetzt ist.

5. Pulverförmige Zusammensetzung nach einem der vorhergehenden Ansprüche, **dadurch gekennzeichnet, dass** die Menge an (b) so ausgewählt ist, dass ein pH von 7,5-10, vorzugsweise 7,5-9,5 und mehr bevorzugt von 7,5-9 nach einer Stunde der Hydratation der Zusammensetzung erhalten wird.

6. Pulverförmige Zusammensetzung nach einem der vorhergehenden Ansprüche, **dadurch gekennzeichnet, dass** das Puffersalz (b) in porösen Granulaten der Komponente (a) und/oder in einer zusätzlichen porösen inerten Phase imprägniert ist.

7. Pulverförmige Zusammensetzung nach einem der vorhergehenden Ansprüche, **dadurch gekennzeichnet, dass** sie auch ein konventionelles Additiv umfasst.

8. Pulverförmige Zusammensetzung nach einem der vorhergehenden Ansprüche, weiter enthaltend pH-empfindliche Medikamente und/oder knochenwachstumsfördernde Mittel, prophylaktische und/oder diagnostische Wirkstoffe, welche danach durch das chemisch gebundene keramische Biomaterial *in vivo* freigesetzt werden sollen, wie zum Beispiel therapeutische Mittel zur Schmerzlinderung, Anästhetika, antiinfektive Mittel und entzündungshemmende Medikamente, Medikamente zur Behandlung von immunologischen Störungen, hormoneller Störung, Gerinnungsverhalten, Krebs und Kombinationen davon.

9. Zusammensetzung, welche nach Hydratation davon ein chemisch gebundenes keramisches Biomaterial zur Verwendung bei endodontischen, orthopädischen und Weichgewebe-Anwendungen bildet, enthaltend (a) eine alkalische Komponente, welche nach Hydratation davon ein chemisch gebundenes keramisches Biomaterial bildet und einen anfänglichen pH von ≥ 10 erzeugt, und (c) eine Hydratationsflüssigkeit, **dadurch gekennzeichnet, dass** die Zusammensetzung auch (b) ein Puffersalz vom Typ [Me:H:Anion] einer ein-, zwei-, drei- oder vierwertigen Mineralsäure in einer Menge von 3-30 Vol.-%, bezogen auf das Gesamtvolumen der Bestandteile im trockenen Zustand, oder 5-20 Gew.-% des Hydratationssystems enthält,
wobei:
Me = Na, K, Mg, Ca;
Anion = CO₃²⁻, PO₄³⁻, SO₃²⁻, SO₄²⁻, SiO₄⁴⁻ oder Fluorid; und
H = ein oder mehrere Wasserstoffionen.

10. Verfahren zur Herstellung der Zusammensetzung nach Anspruch 9, umfassend den Schritt des Vermischens (a) einer alkalischen Komponente, welche nach Hydratation davon ein chemisch gebundenes keramisches Biomaterial bildet und einen anfänglichen pH von ≥ 10 erzeugt, und (c) einer Hydratationsflüssigkeit, **dadurch gekennzeichnet, dass** ein Puffersalz vom Typ [Me:H:Anion] einer ein-, zwei-, drei- oder vierwertigen Mineralsäure in einer Menge von 3-30 Vol.-%, bezogen auf das Gesamtvolumen der Bestandteile im trockenen Zustand, oder 5-20 Gew.-% des Hydratationssystems,
wobei:
Me = Na, K, Mg, Ca;
Anion = CO₃²⁻, PO₄³⁻, SO₃²⁻, SO₄²⁻, SiO₄⁴⁻ oder Fluorid; und
H = ein oder mehrere Wasserstoffionen,
in der Hydratationsflüssigkeit (c) gelöst und/oder der Verbindung (a) zugegeben wird.

## Revendications

1. Composition en poudre formant après hydratation un biomatériau céramique lié chimiquement pour une utilisation dans des applications endodontiques, orthopédiques et de tissus mous, comprenant (a) un composant alcalin qui après hydratation forme un biomatériau céramique lié chimiquement et produit un pH initial de ≥ 10, **caractérisée en ce que** la composition comprend également (b) un sel tampon solide du type [Me:H:Anion] d'un acide minéral mono-, di-, tri- ou tétravalent en une quantité de 3 à 30 % en volume, dans lequel :
Me = Na, K, Mg, Ca ;
Anion = CO₃²⁻, PO₄³⁻, SO₃²⁻, SO₄²⁻, SiO₄⁴⁻ ou fluorure ; et
H = un ou plusieurs ions hydrogène.

2. Composition en poudre selon la revendication 1, **caractérisée en ce que** la phase tampon solide produit les ions choisis parmi HF₂⁻, HCO₃⁻, HPO₄²⁻ et H₂PO₄⁻.

3. Composition en poudre selon la revendication 1 ou 2, **caractérisée en ce que** le composant (a) présente une valeur de pH initial de ≥ 11 après hydratation.

4. Composition en poudre selon l'une quelconque des revendications précédentes, **caractérisée en ce que** le composant (a) est composé d'aluminates et/ou de silicates.

5. Composition en poudre selon l'une quelconque des revendications précédentes, **caractérisée en ce que** la quantité de (b) est choisie de manière à ce qu'un pH de 7,5 à 10, de préférence de 7,5 à 9,5, et plus préférablement de 7,5 à 9 soit obtenu après 1 heure d'hydratation de la composition.

6. Composition en poudre selon l'une quelconque des revendications précédentes, **caractérisée en ce que** le sel tampon (b) est imprégné dans des granules poreuses de composant (a) et/ou dans une phase inerte poreuse additionnelle.

7. Composition en poudre selon l'une quelconque des revendications précédentes, également **caractérisée par le fait qu'**elle comprend un additif classique.

8. Composition en poudre selon l'une quelconque des revendications précédentes, contenant en outre des médicaments sensibles au pH et/ou des agents favorisant la croissance osseuse, des agents ayant une action prophylactique et/ou diagnostique devant être libérés ultérieurement par le biomatériau céramique lié chimiquement *in vivo,* par exemple des agents thérapeutiques destinés au soulagement de la douleur, des anesthésiques, des agents anti-infectieux et des anti-inflammatoires, des médicaments destinés au traitement des troubles immunologiques, des perturbations hormonales, du comportement de la coagulation, du cancer, et de leurs combinaisons.

9. Composition formant après hydratation un biomatériau céramique lié chimiquement pour une utilisation dans des applications endodontiques, orthopédiques et de tissus mous, comprenant (a) un composant alcalin qui après hydratation forme un biomatériau céramique lié chimiquement et produit un pH initial de ≥ 10, et (c) un liquide d'hydratation, **caractérisée en ce que** la composition comprend également (b) un sel tampon du type [Me:H:Anion] d'un acide minéral mono-, di-, tri- ou tétravalent en une quantité de 3 à 30 % en volume par rapport au volume total des constituants à l'état sec, ou de 5 à 20 % en poids du système hydratant,
dans laquelle :
Me = Na, K, Mg, Ca ;
Anion = CO₃²⁻, PO₄³⁻, SO₃²⁻, SO₄²⁻, SiO₄⁴⁻ ou fluorure ; et
H = un ou plusieurs ions hydrogène.

10. Procédé de préparation de la composition selon la revendication 9, comprenant l'étape de mélange (a) d'un composant alcalin qui après hydratation forme un biomatériau céramique lié chimiquement et produit un pH initial de ≥ 10, et (c) d'un liquide d'hydratation, **caractérisé en ce qu'**un sel tampon du type [Me:H:Anion] d'un acide minéral mono-, di-, tri- ou tétravalent en une quantité de 3 à 30 % en volume par rapport au volume total des constituants à l'état sec, ou de 5 à 20 % en poids du système hydratant, dans lequel :
Me = Na, K, Mg, Ca ;
Anion = CO₃²⁻, PO₄³⁻, SO₃²⁻, SO₄²⁻, SiO₄⁴⁻ ou fluorure ; et est
H = un ou plusieurs ions hydrogène
est dissous dans le liquide d'hydratation (c) et/ou ajouté au composé (a).
